(19) Europäisches Patentamt
European Patent Office
Office européen des brevets

(11) Publication number: **0 061 356**
**B1**

(12) **EUROPEAN PATENT SPECIFICATION**

(45) Date of publication of patent specification: **17.07.85**

(51) Int. Cl.[4]: **C 07 K 7/64,** A 61 K 37/02

(21) Application number: **82301555.7**

(22) Date of filing: **24.03.82**

# (54) Antagonists of the antidiuretic and/or vasopressor action of arginine vasopressin.

(30) Priority: **24.03.81 US 247008**
**16.11.81 US 322071**

(43) Date of publication of application:
**29.09.82 Bulletin 82/39**

(45) Publication of the grant of the patent:
**17.07.85 Bulletin 85/29**

(84) Designated Contracting States:
**AT BE CH DE FR GB IT LI LU NL SE**

(56) References cited:
**JOURNAL OF MEDICINAL CHEMISTRY, vol. 25, no. 1, January 1982, Easton, M. MANNING et al. "Design of more potent antagonists of the antidiuretic responses to arginine-vasopressin", pages 45 to 50**
**JOURNAL OF MEDICAL CHEMISTRY, vol. 23, no.4, April 1980 Easton M.KRUSZYNSKI et al."–(B-Mercapto-B,B-Cyclopentamethylenepropionic Acid), 2-(0-Methyl) Tyrosine Arginine-Vaspressin and 1-(B-Mercapto-B, B-Cyclopentamethylenepropionic Acid) Arginine-Vasopressin, Two Highly Potent Antagonists of the Vasopressor Response to Arginine-Vasopressin" pages 364-368**

(73) Proprietor: **Medical College of Ohio**
**3000 Arlington Avenue**
**Toledo Ohio 43699 (US)**

(73) Proprietor: **The Trustees of Columbia University**
**116 Street and Broadway**
**New York, NY 10027 (US)**

(72) Inventor: **Manning, Maurice**
**3741 Driftwood**
**Toledo Ohio 43614 (US)**
Inventor: **Sawyer, Wilbur Henderson**
**12, Warnke Lane**
**Scarsdale New York 105583 (US)**

(74) Representative: **Lamb, John Baxter et al**
**Marks & Clerk 57/60 Lincoln's Inn Fields**
**London WC2A 3LS (GB)**

(56) References cited:
**JOURNAL OF MEDICAL CHEMISTRY, Vol.21, No. 3, March 1978 Easton J. LOWBRIDGE et al. "1-(B-Mercapto-B, B-Cyclopentamethylenepropionic Acid), 4-Valine, -8-D-Arginine Vasopressin, a Potent and Selective Inhibitor of the Vasopressor Response to Arginine-Vasopressin" pages 313-315**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 061 356 B1

**Description**

This invention relates to peptides which antagonize the antidiuretic and/or vasopressor action of arginine vasopressin *in vivo*.

Attempts to develop clinically useful synthetic antagonists of *in vivo* antidiuretic and/or vasopressor responses to arginine vasopressin, the antidiuretic hormone (ADH), have led to the synthesis and pharmacological evaluation of hundreds of analogs of the neurohypophysial peptides, oxytocin and vasopressin.

Analogs which can effectively antagonize *in vivo* vasopressor responses to ADH have been reported by Dyckes et al., *J. Med. Chem.,* vol. 17 (1974), page 250 et seq; Manning et al., *J. Med. Chem.,* vol. 20 (1977), page 1228 et seq; Bankowski et al., *J. Med. Chem.,* vol. 21 (1978), page 850 et seq; Kruszynski et al., *J. Med. Chem.*, vol. 23 (1980), page 364 et seq., and Lowbridge et al., *J. Med. Chem.,* vol. 21 (1978), page 313 et seq.

Krusznski et al. reported that [1-(β-mercapto-β,β-cyclopentamethylenepropionic acid), 2-(O-methyl)-tyrosine]arginine vasopressin and (1-β-mercapto-β,β-cyclopentamethylenepropionic acid]-arginine vasopressin are potent vasopressor antagonists, which also have very low antidiuretic potency.

Manning et al. (1977) described the synthesis of [1-deaminopenicillamine, 4-valine, 8-D-arginine] vasopressin and Lowbridge et al. the synthesis of [1-(β-mercapto-β,β-cyclopentamethylenepropionic acid), 4-valine, 8-D-arginine] vasopressin. Both of these compounds have weak antidiuretic activity and are potent antagonists of the vasopressor response to AVP.

Analogs of vasopressin or oxytocin which antagonize antidiuretic responses to ADH have been reported by Chan et al., *Science,* vol. 161 (1968), page 280 et seq. and *J. Pharmacol. Exp. Ther.,* vol. 174 (1970), page 541 et seq., and vol. 196 (1976), page 746 et seq; Nestor et al., *J. Med. Chem.,* vol. 18 (1975), page 1022 et seq. and Larsson et al., *J. Med. Chem.,* vol 21 (1978) page 746 et seq. None of the compounds reported has been pharmacologically or clinically useful as an antidiuretic antagonist.

The synthesis and evaluation of vasopressin analogs, incorporating etherified tyrosine at the 2-position, valine at the 4-position and D- or L-Arg at the 8-position, which antagonize the anti-diuretic action ADH *in vivo* have been reported by Sawyer et al., *Science,* vol. 212 (1981), page 49 et seq. and by Manning et al., *J. Med. Chem.,* vol. 24 (1981), page 701 et seq.

Synthetic vasopressins have been disclosed in United States Patents Nos. 3,371,080, 3,415,805, 3,418,307, 3,454,549, 3,497,491, and 4,148,787.

Of these, Boissonnnas et al., 3,371,080 discloses that 2-phenylalanine-8-ornithine vasopressin has a vasoconstrictive action equal to that of natural vasopressins but low antidiuretic activity. The remaining references disclose synthetic vasopressins having high or relatively specific antidiuretic activity.

Synthetic modifications of oxytocin are disclosed in United States Patents Nos. 3,691,147 and 3,700,652.

It is therefore apparent that there is a continuing need for the development of pharmacologically and clinically effective antagonists of the antidiuretic action of arginine vasopressin.

It is the object of the invention to provide antagonists to the antidiuretic action of ADH, which are effective *in vivo*. It is a further object to provide antagonists of the vasopressor action of ADH, which have low antidiuretic activity.

According to the invention there are provided compounds of the formula:

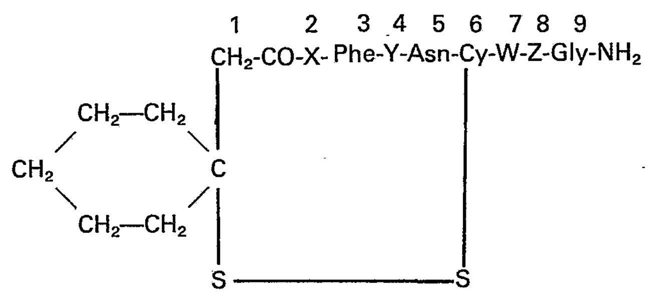

in which:—

X is D-Phe, D-Val, D-Leu, D-Ile, D-Arg, D-Norvaline, D-norleucine, D-cyclohexylalanine, D-α-aminobutyric acid, D-threonine, D-methionine, D-Tyr(H), D- or L-Tyr(X') (in which X' is methyl, ethyl, *n*-propyl, isopropyl or butyl) or Tyr(X'') (in which X'' is H, methyl or ethyl);

Y is Val, when X is other than Tyr(X''), of Gln, when X is Tyr(X'');

W is Pro or, when X is Tyr(X'), $\Delta^3$-Pro; and

Z is D- or L-Arg.

In accordance with one aspect, this invention relates to antagonists of the antidiuretic action of ADH, which are compounds of the formula

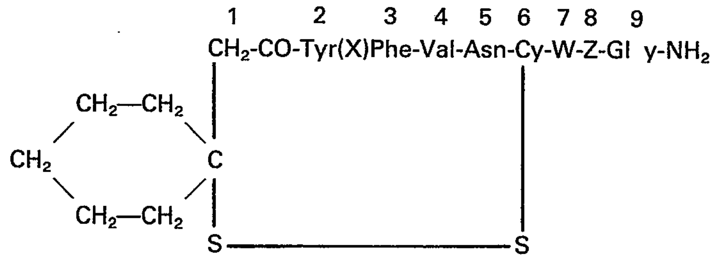

wherein X′ is methyl, ethyl, n-propyl, isopropyl or butyl; Tyr is D- or L-; W is Pro or $\Delta^3$-Pro and Z is L- or D-Arg.

This invention further relates to antagonists of the ADH activity of arginine vasopressin, of the formula

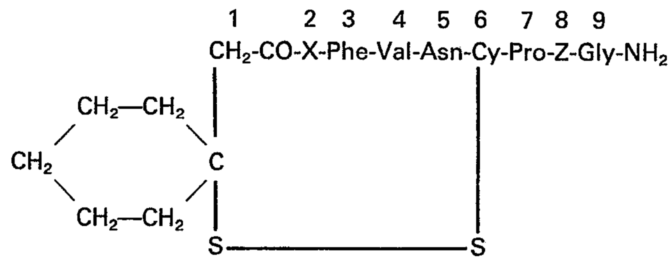

wherein X is D-Tyr(H), D-Phe, D-Val, D-Leu, D-Ile, D-Arg, D-norvaline, D-norleucine, D-cyclohexylalanine, D-α-aminobutyric acid, D-threonine or D-methionine and Z is D- or L-Arg.

This invention further relates to a method for antagonizing the *in vivo* response to ADH, comprising administering to an animal being treated an amount of one of the foregoing compounds, in admixture with a physiologically and pharmaceutically acceptable carrier, effective to antagonize the antidiuretic response to ADH.

In another aspect, this invention relates to antagonists of the vasopressor action of ADH, the compounds being of the formula

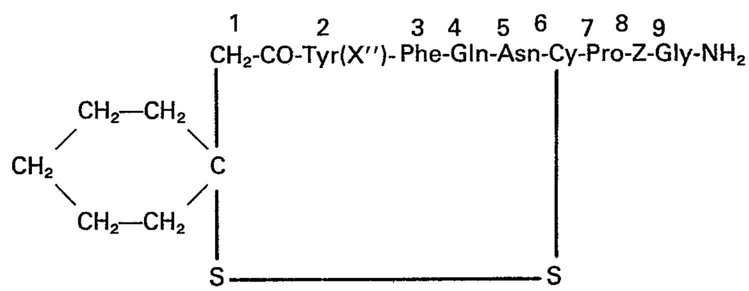

wherein X″ is H, methyl or ethyl and Z is L- or D-Arg. These compounds can be used *in vivo* for antagonizing a vasopressor response to a vasopressor hormone in an animal being treated.

In the following, reference will be made to the accompanying drawings in which:

Figure 1 is a graph showing urine osmolalities, as a function of time, of rats treated intraperitoneally with a compound of this invention; and

Figure 2 is a graph showing urine output, as a function of time, of rats treated with a compound of this invention.

Compounds provided in accordance with the invention are derivatives of arginine vasopressin (AVP). Amino acids are in the L-form unless otherwise indicated. The correlation between full names and abbreviations is: dAVP, 1-deamino-arginine vasopressin; dPAVP, [1-deaminopenicillamine] arginine vasopressin; $d(CH_2)_5AVP$, [1-(β-mercapto-β,β-cyclopentamethylenepropionic acid)]-arginine vasopressin; dVDAVP, 1-deamino[4-valine, 8-D-arginine] vasopressini; dPVDAVP, [1-deamino-penicillamine, 4-valine, 8-D-arginine] vasopressin; $d(CH_2)_5VDAVP$, [1-(β-mercapto-β,β-cyclopentamethylenepropionic acid), 4-valine, 8-D-arginine] vasopressin; dTyr(Me)AVP, 1-deamino[2-(O-methyl)-tyrosine] arginine vasopressin; dPTyr(Me)AVP [1-deaminopenicillamine, 2-(O-methyl)tyrosine] arginine vasopressin; $d(CH_2)_5Tyr(Me)VDAVP$, [1-(β-mercapto-β,β-cyclopentamethylenepropionic acid), 2-O-methyltyrosine, 4-valine, 8-D-arginine] vasopressin; $d(CH_2)_5Tyr(Et)VDAVP$, [1-(β-mercapto-β,β-cyclopentamethylenepropionic acid), 2-O-ethyltyrosine, 4-valine, 8-D-arginine] vasopressin; $d(CH_2)_5Tyr(Me)VAVP$, [1-(β-mercapto-β,β-cyclopentamethylenepropionic acid), 2-(O-methyl)tyrosine, 4-valine]arginine vasopressin; $d(CH_2)_5Tyr(Et)VAVP$, [1-(β-mercapto-β,β-cyclopentamethylenepropionic acid), 2-O-ethyltyrosine, 4-valine] arginine vasopressin; $d(CH_2)_5Tyr(i\text{-}Pr)VDAVP$, [1-(β-mercapto-β,β-cyclopentamethylenepropionic

acid), 2-(O-isopropyl)tyrosine, 4-valine, 8-D-arginine] vasopressin; $d(CH_2)_5Tyr$(n-Pr)-VDAVP, [1-(β-mercapto-β,β-cyclopentamethylenepropionic acid), 2-(O-n-propyl)tyrosine, 4-valine, 8-D-arginine vasopressin; $d(CH_2)_5Tyr$(i-Pr)VAVP, [1-p-mercapto-β,β-cyclopentamethylene-propionic acid), 2-(O-isopropyl)-tyrosine, 4-valine] arginine vasopressin; $d(CH_2)_5Tyr$(n-Pr)VAVP, [1-(β-mercapto-β,β-cyclopentamethylenepropionic acid), 2-(O-n-propyl)tyrosine, 4-valine] arginine vasopressin; and $d(CH_2)_5Tyr$(Et)$V\Delta^3$-$Pro^7$AVP, [1-(β-mercapto-β,β-cyclopentamethylene-propionic acid), 2-(O-ethyl)tyrosine, 4-valine, 7-(3,4-dehydroproline)] arginine vasopressin; $d(CH_2)_5$-D-Tyr VDAVP, (1-β-mercapto-β,β-cyclopentamethylene-propionic acid), 2-D-tyrosine, 4-valine, 8-D-arginine vasopressin; $d(CH_2)_5$D-Tyr VAVP, [1-(β-mercapto-β,β-cyclopentamethylene-propionic acid), 2-D-tyrosine, 4-valine-arginine vasopressin; $d(CH_2)_5$-D-$Phe^2$ VDAVP, [1-(β-mercapto-β,β-cyclopentamethylenepropionic acid), 2-D-phenylalanine, 4-valine, 8-D-arginine] vasopressin; $d(CH_2)_5$D-Phe VAVP, [1(β-mercapto-β,β-cyclopentamethylenepropionic acid), 2-D-phenyl-alanine, 4-valine]-arginine vasopressin; $d(CH_2)_5[Gly^2]$ VAPV, [1-(β[1-(β-mercapto-β,β-cyclopentamethylenepropionic acid), 2-glycine, 4-valine]-arginine vasopressin; $d(CH_2)_5$-[D-$Ala^2$] VAVP, [1-(β-mercapto-β,β-cyclopentamethylenepropionic acid), 2-D-alanine, 4-valine]-arginine vasopressin; $d(CH_2)_5$ [D-$Val^2$] VAVP [1-(β-mercapto-β,β-cyclopentamethylenepropionic acid), 2-D-valine, 4-valine]-arginine vasopressin; $d(CH_2)_5$ [D-$Leu^2$] VAVP, [1-(β-mercapto-β,β-cyclopentamethylenepropionic acid), 2-D-leucine, 4-valine]-arginine vasopressin; $d(CH_2)_5$ [D-$Ile^2$] VAVP, [1-(β-mercapto-β,β-cyclopentamethylenepropionic acid), 2-D-isoleucine, 4-valine]-arginine vasopressin; and $d(CH_2)_5$ [D-$Arg^2$] VAVP, [1-(β-mercapto-β,β-cyclopentamethylenepropionic acid), 2-D-arginine, 4-valine]-arginine vasopressin.

The active peptides were synthesized by solid phase synthesis as described by Bankowsi et al. (1978), *supra*; Merrifield, *J. Am. Chem. Soc.,* vol. 85 (1963), page 2149 et seq. and *Biochemistry,* vol. 3 (1964) page 1385 et seq; Manning, *J. Am. Chem. Soc.,* vol. 90 (1968), page 1348 et seq; Manning et al., *J. Med. Chem.,* vol. 19 (1976) page 376 et seq; Lowbridge et al., *J. Med. Chem.,* vol. 20 (1977) page 1173 et seq; Manning et al., *J. Med. Chem.,* vol. 16 (1973) page 975 et seq; Kruszynski et al. (1980), *supra*; Sawyer et al., (1981), *supra* or Manning et al. (1981) *supra.*

Peptides containing $\Delta^3$-Pro in the 7-position were also prepared in this fashion. Incorporation of $\Delta^3$-Pro into peptides has been described by Felix et al., *J. Peptide Protein Res.,* vol. 10 (1977), page 299 et seq. and Botos et al., *J. Med. Chem.,* vol. 22 (1979), page 926 et seq.

Initial attempts to design an antagonist of the antidiuretic response to arginine vasopressin (AVP) included synthesis of [1-deaminopenicillamine, 4-valine, 8-D-arginine] vasopressin (dPVDAVP) by Manning et al. (1977), *supra,* and of [1-(β-mercapto-β,β-cyclopentamethylenepropionic acid), 4-valine, 8-D-arginine] vasopressin ($d(CH_2)_5$VDAVP), Lowbridge (1978), *supra.* These analogs were designed by replacing the two hydrogens on the β-carbon at the 1-position of the highly active and selective antidiuretic peptide 1-deamino[4-valine, 8-D-arginine] vasopressin (dVDAVP), Manning et al., *J. Med. Chem.,* vol. 16 (1973), page 975 et seq. by two methyl groups and a cyclopentamethylene group, respectively. These substituents had previously been shown to convert the highly potent oxytocic agonist 1-deamino-oxytocin (dOT) into potent antagonists of the oxytocic response to oxytocin, specifically, [1-deaminopenicillamine] oxytocin (dPOT) and [1-(β-mercapto-β,β-cyclopentamethylenepropionic acid)] oxytocin ($d(CH_2)_5$OT). See, Hope et al., *J. Biol. Chem.,* vol 237 (1962) page 1563 et seq.; Schulj et al. *J. Med. Chem.,* vol. 9 (1966) page 647 et seq. and Nestor et al., *J. Med. Chem.,* vol. 18 (1975), page 284 et seq.

Surprisingly, neither dPVDAVP nor $d(CH_2)_5$VDAVP was an antagonist of the antidiuretic response to AVP although possessing 0.1 and only 0.0001 times the antidiuretic activity of dVDAVP, respectively. Each, however, was a potent antagonist of the vasopressor response to AVP, expressed as $pA_2$. $pA_2$ represents the negative logarithm to the base 10 of the average molar concentrations of antagonist which will reduce the specific biological response to 2x units of an agonist to the level of response to x units of the agonist. dPVDAVP and $d(CH_2)_5$VDAVP had antivasopressor $pA_2$ values of 7.82 and 7.68, respectively.

The discovery of these two vasopressor antagonists dPVDAVP and $d(CH_2)_5$VDAVP led to exploration of the effects of β,β-dimethyl and β,β-cyclopentamethylene substitutions at the 1-position in other analogs of AVP, particularly in combination with the substitution of O-methyltyrosine at the 2-position of the highly active antidiuretic and vasopressor agonist 1-deamino-arginine vasopressin (dAVP) in hopes of obtaining an antivasopressor peptide even more potent and selective than dPVDAVP or $d(CH_2)_5$VDAVP. See, Huguenin et al., *Helv. Chem. Acta.,*vol. 49 (1966), page 695 et seq; Manning et al., *J. Med. Chem.,* vol 19 (1976), page 842 et seq., and Law et al., *J. Am. Chem. Soc.,* vol. 82 (1960) page 4579 et seq.

The discovery of the antidiuretic antagonists $d(CH_2)_5Tyr$(alk)VAVP Sawyer et al., (1981), *supra,* Manning et al., (1981) *supra,* led to the synthesis of other position two substituted analogs. Enhanced anti-antidiuretic potencies were exhibited by the various O-alkyl D-tyrosine analogs, Manning et al., in *Peptides, Structure, Function,* Dan H. Rich and E. Gross, eds., Pierce Chemical Co., (1981) p. 257 et seq, and J. Med. Chem. Vol. 25 (1982) p. 45 et seq. The unalkylated D-tyrosine isomers of d(CH2)$_5$VDAVP and $d(CH_2)_5$VAVP, i.e., $d(CH_2)_5$D-Tyr-VDAVP and $d(CH_2)_5$-D-Tyr-VAVP were also shown to be anti-antidiuretics. Attempts to further enhance anti-antidiuretic potency and selectivity have led to the synthesis of analogs of $d(CH_2)_5$D-$Tyr^2$VAVP and $d(CH_2)_5$D-$Tyr^2$VDAVP containing other D-amino acids in place of D-tyrosine at position two, in accordance with the present invention.

It was surprisingly found by Bankowski et al. (1978), *supra,* that of [1-deaminopenicillamine]-arginine vasopressin (dPAVP) and [1-deaminopenicillamine, 2-(O-methyl)tyrosine] arginine vasopressin

(dPTyr(Me)-AVP), dPAVP was less potent than either dPVDAVP or $d(CH_2)_5$VDAVP but dPTyr(Me)AVP had an antivasopressor $pA_2$ of 7.96 and was the most potent antivasopressor peptide then known.

The effect, on antivasopressor potency of combining the β,β-cyclopentamethylene and O-methyltyrosine substitutions in dAVP was developed in accordance with this invention to provide [1-(β-mercapto-β,β-cyclopentamethylenepropionic acid), 2-(O-methyl)-tyrosine]-arginine vasopressin ($d(CH_2)_5$Tyr(Me)AVP) of the structure

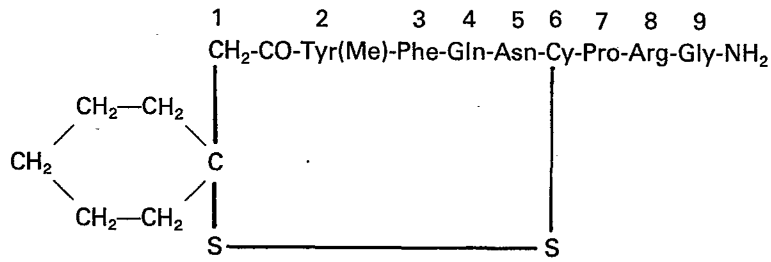

This compound had very high antivasopressor potency and very weak antidiuretic activity, as did the unmethylated 2-tyrosine derivative $d(CH_2)_5$AVP.

The compounds of this invention which have activity as antagonists of the antidiuretic activity of arginine vasopressin belong to the 4-valine-8-arginine vasopressin series and are of Formula I

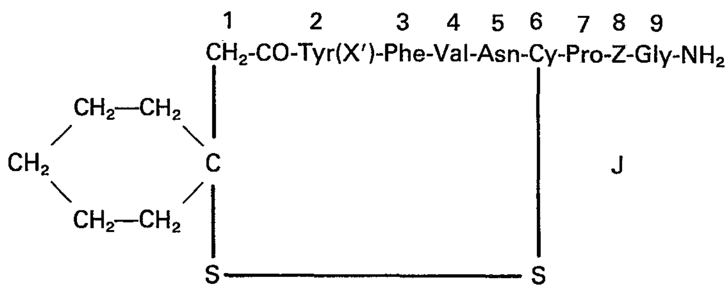

J

wherein Tyr is D- or L- and X′ and Z are:

| X′ | Z |
|---|---|
| Me | D-Arg |
| Et | D-Arg |
| Me | L-Arg |
| Et | L-Arg |
| i-Pr | D-Arg |
| n-Pr | D-Arg |
| i-Pr | L-Arg |
| Bu | L- or D-Arg |

A related series of compounds, referred to herein as compounds, of Formula II, are as above, wherein Pro at the 7-position is replaced by $\Delta^3$-Pro.

The compounds of Formula I are related to a previously-reported antagonist of vasopressor responses to ADH, [1-(β-mercapto-β,β-cyclopentamethylenepropionic acid), 4-valine, 8-D-arginine] vasopressin $d(CH_2)_5$VDAVP, Lowbridge et al. (1978). Although not an antagonist of antidiuretic responses to ADH *in vivo*, this analog was a competitive antagonist of the activation of renal medullary adenylate cyclase by ADH *in vitro*, Butlen et al., *Mol. Pharmacol.,* vol. 14 (1978), page 1006, et seq. The work of Larsson et al. (1978), *supra*, also indicated the feasibility of making O-alkyl-tyrosine substitutions to convert this type of peptide into an antagonist of the antidiuretic response *in vivo*.

Compounds of Formula II contain a $\Delta^3$-$Pro^7$ unit thought by Botos et al., *supra*, to contribute to high antidiuretic acitivity of certain AVP analogues.

As shown by intraperitoneal administration of these compounds of the invention to normally-hydrated conscious rats, (O-ethyl)-tyrosine substitution at the 2-position in compounds of Formula I is more effective

than (O-methyl)tyrosine substitution. The (O-propyl)-tyrosine compounds of Formula I also have impressive anti-ADH activity. However a 2-(O-Et)-tyrosine compound of Formula II is the most effective anti-ADH compound evaluated to present. The 8-L-arginine analogs are more potent than the corresponding 8-D-arginine analogs.

It appears that higher doses of $d(CH_2)_5Tyr(Et)VAVP$ almost completely block the antidiuretic action of endogenous ADH. For example, the 30 μg/kg dose of $d(CH_2)_5Tyr(Et)VAVP$ raised urine flow to a mean of 27 ml/kg per hr during the second hour after injection. Spontaneous urine flow in female rats homozygous for the Brattleboro strain that secrete no ADH at all averages 32 ml/kg per hr, Sawyer, et al., *Endocrinology,* vol. 95 (1974), page 140 et seq.

The importance of minor structural modifications is indicated by findings that corresponding β,β-diethyl and β,β-dimethyl analogs of $d(CH_2)_5Tyr(Et)VAVP$ do not exert detectable antagonist activity in the intravenous rat antidiuretic assay. The presence of the 4-valine also contributes to antagonist activity. Substitution of a 4-glutamine unit in $d(CH_2)_5Tyr(Et)VAVP$ results in loss of antagonist acitivity.

Compounds of the invention having Gln in the 4-position, which antagonize the vasopresser response to AVP, are useful in pharmacological studies on the role of AVP in regulating blood pressure under normal and pathophysiological conditions. Clinical applications include use as diagnostic and therapeutic antihypertensive agents. For therapeutic purposes, these compounds will be used in the same fashion as the known antihypertensive drug Captopril, D. B. Case et al., "Progress in Cardiovascular Diseases," vol. 21 (1978), page 195 et seq.

The compounds of Formulas I and II are very effective antagonists of the antidiuretic response to ADH. They can therefore be used in pharmacological studies on the contribution of ADH to a variety of pathological states involving water retention. It is further contemplated that they could be effective and specific agents for treating the syndrome of inapropriate secretion of ADH, that is, the Schwartz-Bartter syndrome or SIADH. This syndrome can complicate a number of disorders, including carcinomas, pulmonary diseases, intracranial diseases and head injuries, Bartter et al., *Am. J. Med.,* vol. 42 (1967), page 790 et seq.

It was found, in accordance with the present invention, that some $d(CH_2)_5VAVP$ derivatives having a D-amino acid other than tyrosine and larger than alanine in the 2-position are more potent antagonists of the antidiuretic action of AVP than compounds having D- or tyrosine ether units or a D-tyrosine unit at the 2-position of $d(CH_2)_5VAVP$ or $d(CH_2)_5VDAVP$.

Preferred compounds of this invention are those wherein the 8-substituent is Arg or the 2-substituent is D-Phe, D-Val, D-Leu and D-Ile.

As shown by intravenous administration of the compounds of the invention to normally-hydrated conscious rats and to hydrated rats anesthetized with ethanol, compounds having D-Phe, D-Val, D-Leu or D-Ile substituents at the 2-position have high $pA_2$ values and effective doses near or lower than the lowest effective doses known heretofore.

Compounds having D-Phe, D-Val, D-Leu or D-Ile at the 2-position and Arg at the 8-position are also pure antidiuretic antagonists, i.e. these compounds have no transient antidiuretic agonism. Moreover, these compounds are more selective in their activity, by virtue of high anti-ADH/antivasopressor activity ratios, than known compounds.

The compounds of this invention can be employed in mixture with conventional excipients, i.e., physiologically and pharmaceutically acceptable organic or inorganic carriers suitable for parenteral or enteral application, which do not interact deleteriously with the active compounds. Accordingly, the present invention also provides pharmaceutical compositions comprising a compound in accordance with the invention in association with a pharmaceutical carrier or diluent.

Suitable pharmaceutically acceptable carriers include, but are not limited to, water, salt solutions, alcohols, vegetable oils, polyethylene glycols, gelatine, lactose, amylose, magnesium stearate, talc, silicic acid, viscous paraffin, perfume oil, fatty acid monoglycerides and diglycerides, pentaerythritol fatty acid esters, hydroxy-methylcellulose, polyvinyl pyrrolidone, etc. The pharmaceutical preparations can be sterilized and if desired mixed with auxiliary agents, e.g., lubricants, preservatives, stabilizers, wetting agents, emulsifiers, salts for influencing osmostic pressure, buffers, colouring, flavoring and/or aromatic substances and the like which do not deleteriously react with the active compounds.

For parenteral or intranasal application, solutions, preferably aqueous solutions, as well as suspensions, emulsions, or implants, including suppositories, are particularly suitable. Ampoules are convenient unit dosages.

The compounds of this invention are generally administered to animals, including but not limited to mammals, e.g., livestock, household pets, humans, cattle, cats and dogs. A diuretically effective daily dosage of the active compounds can be administered parenterally in a single dosage or as divided dosages throughout the day.

Parenteral or intranasal administration is preferred, the anti-antidiuretic compounds of this invention being particularly valuable in the treatment of humans afflicted with water retention of any etiology. In this regard, they can be administered in substantially the same manner as the known compounds oxytocin and vasopressin, to achieve their physiological effects.

It will be appreciated that the actual preferred amounts of active compounds used will vary according to the specific compound being utilized, the particular compositions formulated, the mode of application,

and the particular organism being treated. Optimal application rates under/in a given set of conditions can be ascertained by those skilled in the art of using conventional dosage determination tests in view of the above guidelines.

Preferred antidiuretic antagonists of the invention are [1-(β-mercapto-β,β-cyclopentamethylene-propionic acid), 2-(O-ethyl)tyrosine, 4-valine]-arginine vasopressin, most preferably the 8-L-arginine compound. Also preferred is a corresponding $\Delta^3$-$Pro^7$ compound.

Other preferred compounds are those wherein X is D-Phe, D-Val, D-Leu or D-Ile and Z is L-Arg. The D-Ile or D-Phe compound is most preferred.

In order that the invention may be well understood the following examples are given by way of illustration only. In the examples, unless otherwise indicated, all parts and percentages are by weight.

Chloromethylated resin (Bio-Rad® Bio-Beads® 5X-1) was esterified by the procedure of Gisin, *Helv. Chim. Acta,* vol. 56 (1973), page 1476 et seq. with Boc-Gly until 0.47 mmol/g and 0.64 mmol/g were incorporated. Amino acid derivatives including Boc-Tyr(Me) (Rf (A) 0.7; Rf (B) 0.8, were supplied by Bachem Inc., or synthesized.

Triethylamine (TEA) and N-methylmorpholine (NMM) were distilled from ninhydrin.

Acetic acid used as the HCl-acetic acid cleavage reagent was heated under reflux with boron triacetate and distilled from the reagent. Dimethylformamide (DMF) was distilled under reduced pressure immediately before use. Methanol was dried with magnesium methoxide and distilled. Other solvents and reagents were of analytical grade.

Thin layer chromatography (TLC) done on silica gel plates (0.25 mm, Brinkman Silplate) using the following solvent systems: A. cyclohexane-chloroform-acetic acid (2:8:1 v/v); B. propan-1-ol-ammonia (34%) (2:1 v/v); C. ethanol (95%)-ammonia (34%) (3:1 v/v); D. chloroform-methanol (7:3 vv); E. butan-1-ol-acetic acid-water (4:1:5 v/v, upper phase); F. butan-1-ol-acetic acid-water-pyridine (15:3:3:10 v/v). The applied loadings were 10—50 µg. The minimum length of the chromatograms was 10 cm. Chloroplatinate reagent and iodine vapor were used for development of the chromatograms.

Amino acid analysis of the peptides was done by the method of Spackman et al., *Anal. Chem.,* vol. 30 (1958), page 1190 et seq, in which peptide samples weighing about 0.5 mg were hydrolyzed with constant boiling hydrochloric acid (400 µl) in evacuated and sealed ampules for 18 h at 120°C. The analyses were performed using a Beckman Automatic Amino Acid Analyzer, Model 121. Molar ratios were referred to Gly=1.00. Elemental analyses were performed by Galbraith Laboratories, Inc., Knoxville, Tenn. The analytical results for the elements indicated by their respective symbols were within ±0.4% of theoretical values. Optical rotations were measured with a Bellingham Stanley, Ltd., Model A polarimeter, type pl.

Example 1

β-(S-benzylmercapto)-β,β-cyclopentamethylenepropionyl-Tyr(Me)-Phe-Gln-Asn-Cys(Bzl)-Pro-Arg(Tos)-Gly-$NH_2$

(a) Combination of solid phase and solution methods

Boc-Tyr(Me)-Phe-Gln-Asn-Cys(Bzl)-Pro-Arg(Tos)-Gly-$NH_2$, prepared by the method of Bankowski et al., *J. Med. Chem.,* vol. 21 (1976), page 842 et seq., (319 mg, 0.26 mmol), was dissolved in TEA (6.5 ml) and stirred at room temperature for 40 mins. Cold ether (20 ml) was added to produce a precipitate which was filtered and washed with ether (5×10 ml). The product was dried *in vacuo* over sodium hydroxide pellets. This material (318.5 mg) was dissolved in DMF (0.8 ml), to which was added N-methylmorpholine (10 µl). The resulting solution had a pH of 7—8, measured with moist pH paper. After this neutralized solution was stirred at room temperature for 30 min, a solution of *p*-nitrophenyl β-(S-benzyl-mercapto)-β,β-cyclopentamethylenepropionate, Nestor et al., *J. Med. Chem.* vol. 18 (1975) page 284 et seq., (445 mg, 1.155 mmol in 0.4 ml of DMF) was added. The reaction mixture was stirred at room temperature. After 72 hours' stirring, TLC analysis using system D showed that the reaction mixture still contained a trace of the free octapeptide amide. N-Hydroxybenzotriazole monohydrate, Konig et al., *Chem. Ber.,* vol. 103 (1970), page 788 et seq., (39.3 mg, 0.26 mmol) was added. Coupling was complete within 5 hours. The precipitate was filtered, washed with cold ethyl acetate (4×10 ml) and dried *in vacuo.* The crude product (330 mg) was twice reprecipitated from DMF-methanol to give the acylpeptide amide (295.2 mg, 77.3%): mp. 209—211°C; $[\alpha]_D^{24}=-43.6°$ (C 0.5, DMF): $R_f$(E) 0.45, $R_f$ (F) 0.63 Anal. ($C_{73}H_{94}O_{14}S_3$) C, H, N.

Amino acid analysis: Tyr, 0.80; Phe, 1.01; Glu, 1.04; Asp, 1.02; Cys(Bzl), 0.98; Pro, 1.06; Arg, 1.01; Gly, 1.00; $NH_3$ 2.91.

(b) Total synthesis on resin

Bos-Tyr(Me)-Phe-Gln-Asn-Cys(Bzl)-Pro-Arg(Tos)-Gly-resin (1.11 g, 0.4 mmol prepared from Boc-Gly-resin using solid phase methodology) was converted to the acyloctapeptide resin (1.167 g, weight gain 57 mg, 97.6% of theory) in one cycle of deprotection, neutralization and coupling with *p*-nitrophenyl-β-(S-benzylmercapto)-β,β-cyclopentamethylenepropionate, see Nestor *supra.* The resin was ammonolyzed, Manning, *J. Am. Chem. Soc.,* vol. 90 (1968), page 1348 et seq. The product was extracted with dimethylformamide (DMF). After the solvent was evaporated *in vacuo,* the residue was precipitated by addition of water. The crude product (410 mg) was twice reprecipitated from DMF-ethanol to give the acyloctapeptide (302 mg, 50.7% based upon initial glycine content of the resin); mp. 206—208°C (decomp);

$R_f$ (E), 0.45, $R_f$ (F) 0.63; $[\alpha]_D^{24}=-43.1°$ (C 1, DMF).

Anal. ($C_{73}H_{94}N_{14}S_3$) C, H, N.

Amino acid analysis: Tyr, 0.79; Phe, 1.01; Glu, 1.03; Asp, 1.04; Cys(Bzl), 0.97; Pro, 1.03; Arg, 0.99; Gly 1.00; $NH_3$, 2.95.

Example 2

β-(S-Benzylmercapto)-β,β-cyclopentamethylenepropionyl-Tyr(Bzl)-Phe-Gln-Asn-Cys(Bzl)-Pro-Arg(Tos)-Gly-$NH_2$.

Boc-Tyr(Bzl)-Phe-Gln-Asn-Cys(Bzl)-Pro-Arg(Tos)Gly-resin (1.46 g, 0.5 mmol) was converted to the acyloctapeptide resin (1.55 g, weight gain 70 mg, 95.9% of theory) as in Example 1 by one cycle of deprotection, neutralization and coupling with *p*-nitrophenyl β-(s-benzylmercapto)-β,β-cyclopentamethylenepropionate. The product obtained by ammonolysis of the resin was extracted with DMF. The solvent was evaporated *in vacuo* and the residue was precipitated by the addition of water. The crude product (723 mg) was reprecipitated from DMF-ethanol and DMF-2% aqueous AcOH. (488 mg; 62.4% based on initial Gly content on the resin); mp. 183—185°C; $R_f$ (E) 0.38; $R_f$ (D) 0.41; $[\alpha]_D^{23}=-32.9°$ (C 1 DMF).

Anal. ($C_{79}H_{98}N_{14}O_{14}S_3$) C, H, N.

Amino acid analysis: Tyr, 0.97; Phe, 1.02; Glu, 1.05; Asp, 1.01; Cys(Bzl), 0.98; Pro, 1.04; Arg, 0.98; Gly, 1.00; $NH_3$.

Example 3

[1-(β-mercapto-β,β-cyclopentamethylenepropionic acid), 2-(O-methyl)tyrosine] arginine vasopressin.

(a) From nonapeptide amide

A solution of the protected nonapeptide amide, prepared as in Example 1, (170 mg, 0.114 mmol) in 400 ml of ammonia (dried over sodium and redistilled) was stirred at the boiling point with sodium from a stick of the metal contained in a small bore glass tube until a light blue colour persisted in the solution for 30 sec., in accordance with duVigneaud, *J. Am. Chem. Soc.*, vol. 76 (1959), page 3115 et seq. Dry glacial acetic acid (0.4 ml) was added to discharge the colour. The solution was evaporated. A solution of the residue in aqueous acetic acid (0.2%; 800 ml), was treated with 2M ammonium hydroxide solution to give a solution of pH 7.5. To this stirred solution was added gradually an excess of a solution of potassium ferricyanide (0.01M, 11.4 ml), Hope et al., *J. Biol. Chem.*, vol. 237 (1962), page 1563 et seq. The yellow solution was stirred for 90 min more and for 1 hour with anion-exchange resin (BioRad® AG-3, C1-form, 10 g damp weight). The suspension was filtered slowly through a bed of resin (80 g damp weight). The resin bed was washed with 300 ml of aqueous 0.2% acetic acid and the combined filtrate and washings were lyophylized. The resulting powder (1386 mg) was desalted on a Sephadex G-15 column (110×2.7 cm) and eluted with aqueous acetic acid (50%) at a flow rate 4 ml/h by the technique of Manning et al., *J. Chromatog.*, vol. 38 (1968), page 396 et seq. The eluate was fractioned and monitored for absorbance of 280 nm. The fractions comprising the major peak were pooled and lyophilized. The residue (55.5 mg) was further subjected to gel filtration on a Sephadex G-15 column (100×1.5 cm) and eluted with aqueous acetic acid (0.2M) at a flow rate of 2.5 ml/h. The peptide was eluted in a single peak (absorbance 280 nm). Lyophilization of the pertinent fractions yielded the vasopressin analog (49 mg, 37.3%) $R_f$(E) 0.19; $R_f$(F) 0.30; $[\alpha]_D^{22}-59.6$ (c 0.19, 1M AcOH).

Amino acid analysis: Tyr, 0.81; Phe 1.01; Glu, 1.04; Asp, 0.98; Pro, 1.04; Arg, 0.95; Gly, 1.00; $NH_3$, 3.10. Analysis following performic acid oxidation prior to hydrolysis according to Moore, *J. Biol. Chem.*, vol. 238 (1963), page 235 et seq., gave a Cys($O_3$H)-Gly ratio of 1.03:1.00.

(b) From acyloctapeptide

Treatment of the acyloctapeptide (160 mg, 0.107 mmol) as described in Example 3 (a) yielded the analog (64 mg, 51.7%), which was indistinguishable from the foregoing preparation by TLC: $[\alpha]_D^{23}$ −59.1° (C 0.5, 1M AcOH).

Amino acid analysis: Tyr, 0.80; Phe, 1.02; Glu, 1.02; Asp, 0.98; Pro, 1.03; Arg, 0.96; Gly, 1.00; $NH_3$, 3.05. Analysis following performic acid oxidation prior to hydrolysis gave a Cys-($O_3$H)-Gly ratio of 1.02:1.00.

Example 4

[1-(β-mercapto-β,β-cyclopentamethylenepropionic acid)]arginine vasopressin

Treatment of the acyloctapeptide (173 mg, 0.111 mmol) as described in Example 3 (a) yielded the analog (66 mg, 52.5%) $R_f$(E) 0.19, $R_f$(F) 0.43. $[\alpha]_D^{23}$ −58.7° (C 0.5, 1M AcOH).

Amino acid analysis: Tyr, 0.96; Phe, 0.98; Glu, 1.01; Asp, 1.01; Pro, 1.05; Gly, 1.00; $NH_3$, 2.95. Analysis following performic acid oxidation prior to hydrolysis gave a Cys($O_3$H)-Gly ratio of 1.01:1.00.

Example 5

[1-(β-mercapto-β,β-cyclopentamethylenepropionic acid), 2-(O-alkyl)tyrosine, 4-valine]-(L- and D-)-arginine vasopressin.

Compounds of this series were prepared by solid-phase synthesis, modified as in Manning et al., *J. Med. Chem.*, vol. 16 (1973), page 975 et seq. and Kruszynski et al., *J. Med. Chem.*, vol. 23 (1980) page 364 et seq., to obtain protected intermediates for each analog. The procedures of Bodanszky et al., *J. Am. Chem.*

*Soc.*, vol. 81 (1959), page 5688 et seq. and *J. Org. Chem.*, vol. 39 (1974) page 444 et seq., employing a *p*-nitrophenyl ester, facilitated by the use of hydroxybenzotriazole (Konig et al., *supra*), were used for the coupling of β-(S-benzylmercapto-β,β-cyclopentamethylenepropionic acid in accordance with Nestor, *supra*, to obtain precursor compounds. Each precursor was deblocked (duVigneaud, *supra*) with sodium in liquid ammonia. The resulting disulphhydryl compounds were oxidatively cyclized with potassium ferricyanide (Hope et al., *supra*). The analogs were desalted and purified by gel filtration on Sephadex® G-15 by a two step procedure using 50% acetic acid and 0.2M acetic acid, respectively, as eluants. The purity and identity of each analog was ascertained by thin-layer chromatography in three different solvent systems, Kruszynski et al., *J. Med. Chem.*, vol. 23 (1980), page 364 et seq., and by amino acid analysis as above.

Boc-Phe-Val-Asn-Cys(Bzl)-Pro-D-Arg(Tos)-Gly-resin

Boc-Gly-resin (1.562 g, 1.0 mmol, of Gly) was subjected to six cycles of deprotection, neutalization, and coupling to yield the protected heptapeptidyl resin A (2.522 g, 1.0 mmol).

Boc-Phe-Val-Asn-Cys(Bzl)-Pro-Arg(Tos)-Gly-resin

The protected heptapeptidyl resin B (2.522 g, 1.0 mmol) was prepared from 1.562 g (1.0 mmol) of Boc-Gly-resin using solid-phase methodology.

Boc-Tyr(Me)-Phe-Val-Asn-Cys(Bzl)-Pro-D-Arg(Tos)-Gly-resin

A single cycle of solid-phase peptide synthesis with Boc-Tyr(me) as the carboxy component converted heptapeptidyl resin A (1.261 g, 0.5 mmol) to the corresponding tert-butyloxycarbonyloctapeptidyl resin C (1.35 g, 0.5 mmol).

Boc-Tyr(Et)-Phe-Val-Asn-Cys(Bzl)-Pro-D-Arg(Tos)-Gly-resin

The heptapeptidyl resin A (1.261 g, 0.5 mmol) yielded the tert-butyloxycarbonyloctapeptidyl resin D (1.357 g, 0.5 mmol) in one cycle of solid-phase peptide synethsis with Boc-Tyr(Et) as the carboxy component.

Boc-Tyr(Me)-Phe-Val-Asn-Cys(Bzl)-Pro-Arg(Tos)-Gly-resin

The heptapeptidyl resin B (1.261 g, 0.5 mmol) was converted to protect octapeptidyl resin E (1.35 g, 0.5 mmol) in one cycle of deprotection, neutralization and coupling with Boc-Tyr(Me).

β-(S-benzylmercapto)-β,β-cyclopentamethylenepropiony-Tyr(Et)-Phe-Val-Asn-Cys(Bzl)-Pro-Arg(Tos)-Gly-resin

The heptapeptidyl resin B (1.261 g, 0.5 mmol) was converted to the acyloctapeptide resin (1.43 g, 0.5 mmol) in two cycles of solid phase peptide synthesis using as the carboxy component, respectively: Boc-Tyr(Et) and *p*-nitrophenyl β-(S-benzylmercapto)-β,β-cyclopentamethylenepropionate.

Boc-Tyr(Me)-Phe-Val-Asn-Cys(Bzl)-Pro-D-Arg(Tos)-Gly-$NH_2$

The protected octapeptide resin C (1.35 g, mmol) was ammonolyzed and the product extracted with warm DMF. The product was precipitated by addition of water. The crude product was reprecipitated from DMF-ethanol-ethyl ether to give the pure product as a white powder (0.581 g, 88.52% based on initial Gly content of the resin) mp. 239—240°C; $[\alpha]_D^{24}=-14.9°$ (C=1, DMF); $R_f$ (E), 0.54, $R_f$(D), 0.73; Anal. ($C_{63}H_{85}N_{13}O_{14}S_2$) C, H, N.

Amino acid analysis: Tyr, 1.02; Phe, 0.98; Val, 1.02; Asp 1.00; Cys(Bzl), 0.98; Pro, 1.01; Arg, 0.97; Gly, 1.00; $NH_3$, 2.1.

Boc-Tyr(Et)-Phe-Val-Asn-Cys(Bzl)-Pro-D-Arg(Tos)-Gly-$NH_2$

Treatment of the protected octapeptide resin D (1.357 g, 0.5 mmol) as above yielded the Boc-octapeptideamide (0.535 g, 80.69% based on initial Gly content of the resin) mp. 211—213°C; $[\alpha]_D^{24}=-16.4°$ (C=1, DMF); $R_f$(E), 0.61, $R_f$(D), 0.83; Anal. ($C_{64}H_{87}N_{13}O_{14}S_2$) C, H, N.

Amino acid analysis: Tyr, 0.99; Phe, 1.00; Val, 1.01; Asp, 1.02; Cys(Bzl), 0.98; Pro, 1.00; Arg, 0.98; Gly, 1.00; $NH_3$, 2.13.

Boc-Tyr(Me)-Phe-Val-Asn-Cys(Bzl)-Pro-Arg(Tos)-Gly-$NH_2$

Treatment of the protected octapeptide resin E (1.35 g, 0.55 mmol) as above gave the corresponding Boc-octapeptideamide (0.597 g, 90.96% based on the initial Gly content of the resin). mp. 216—217°C decomp.);

$[\alpha]_D^{24}=-34.82°$ (C=1, DMF); $R_f$(E), 0.54, $R_f$(D), 0.73; Anal. ($C_{63}H_{85}N_{13}O_{14}S_2$) C, H, N.

Amino acid analysis: Tyr, 0.99; Phe, 1.00; Val, 1.02; Asp, 1.01; Cys(Bzl), 0.98; Pro, 1.01, Arg, 0.98; Gly, 1.00; $NH_3$, 2.09.

β-(S-benzylmercapto-β,β-cyclopentamethylenepropionyl-Tyr(Et)-Phe-Val-Asn-Cys(Bzl)-Pro-Arg(Tos)-Gly-$NH_2$

The protected acyloctapeptide resin (1.43 g, 0.5 mmol) was ammolyzed and the product extracted with

warm DMF. The product was precipitated by addition of water. The crude product was recprecipitated from DMF-ethanol-ethyl ether to give the pure product (0.490 g, 66.54% based on initial Gly content of the resin). mp. 211—213°C; $[\alpha]_D^{24}=-39.8°$ (C=1, DMF); $R_f$(E), 0.59, $R_f$(D), 0.75; Anal. ($C_{74}H_{97}H_{13}O_{13}S_3$) C, H, N.

Amino acid analysis: Tyr, 0.99; Phe, 1.01; Val, 1.01; Asp, 1.01; Cys(Bzl), 0.99; Pro, 1.02; Arg, 0.98; Gly, 1.00; $NH_3$, 2.07.

β-(S-benzylmercapto)-β,β-cyclopentamethylenepropionyl-Tyr(Me)-Phe-Val-Asn-Cys(Bzl)-Pro-D-Arg(Tos)Gly-$NH_2$

The tert-butyloxycarbonyloctapeptide amide prepared above (0.270 g, 0.206 mmol) was dissolved in TFA (3 ml) and to stand at room temperature of 20 min. Cold ether was added. The precipitated material was filtered and washed with ether (5×10 ml). The product was dried *in vacuo* over sodium hydroxide pellets. This material (250 mg) was dissolved in DMF (0.8 ml) to which solution N-methylmorpholine was added to give a solution of pH 7—8 (moist pH paper). The neutralized solution was stirred at room temperature for 20 min. A solution of *p*-nitrophenyl β-(S-benzylmercapto-β,β-cyclopentamethylenepropionate (0.135 g, 0.37 mmol) and N-hydroxybenzo-triazole monohydrate (57 mg, 0.37 mmol) in DMF (1.0 ml) was added. The reaction mixture was stirred at room temperature overnight and TLC (system E) showed that the reaction was complete. Methanol (80 ml) and ether (20 ml) were added with vigorous mixing. The precipitated material was filtered, washed with a mixture of methanol-ether (8:2), and dried *in vacuo*. The crude product (270 mg) was re-precipitated from DMF-methanol to give the acyl peptide amide (263 mg, 75.2%); mp. 220—221°C; $[\alpha]_D^{24}=-25.7°$ (C=1, DMF); $R_f$(E), 0.55, $R_f$(D), 0.83; Anal. ($C_{73}H_{95}N_{13}O_{13}S_3$) C, H, N.

Amino acid analysis: Tyr, 0.98; Phe, 1.01; Val, 1.02; Asp, 1.02; Cys(Bzl), 0.97; Pro, 1.03; Arg, 1.0; Gly, 1.00; $NH_3$, 2.06.

β-(S-Benzylmercapto-β,β-cyclopentamethylenepropionyl-Tyr(Et)-Phe-Val-Asn-Cys(Bzl)-Pro-D-Arg(Tos)-Gly-$NH_2$

The tert-butyloxycarbonyloctapeptide amide (0.398 g, 0.3 mmol) was deprotected and coupled with *p*-nitrophenyl β-(S-benzylmercapto-β,β-cyclopentamethylenepropionate (0.232 g, 0.6 mmol) as described above to give the acyloctapeptide amide (0.361 g, 81.67%) mp. 222—224° C; $[\alpha]_D^{20}=-22.8°$ (C=0.5, DMF); $R_f$(E), 0.5, $R_f$(D), 0.83; Anal. ($C_{74}H_{97}N_{13}O_{13}S_3$) C, H, N.

Amino acid analysis: Tyr, 1.0; Phe, 1.02; Val, 1.03; Asp, 1.02; Cys(Bzl), 0.98; Pro, 1.03; Arg, 0.99; Gly, 1.00; $NH_3$, 2.11.

β-(S-benzylmercapto)-β,β-cyclopentamethylenepropionyl-Tyr(Me)-Phe-Val-Asn-Cys(Bzl)-Pro-Arg(Tos)-Gly-$NH_2$

Tert-butyloxycarbonyloctapeptide amide (0.394 g, 0.3 mmol) was deprotected and coupled with p-nitrophenyl β-(S-benzylmercapto)-β,β-cyclopentamethylenepropionate (0.232 g, 0.6 mmol) as above to produce the acyloctapeptide amide (0.388 g, 88.65%); mp. 211—214°C; $[\alpha]_D^{21}=-39.2°$ (C=1, DMF); $R_f$(E), 0.47, $R_f$(D), 0.85; Anal. ($C_{73}H_{95}N_{13}O_{13}S_3$) C, H, N.

Amino acid analysis: Tyr, 0.99; Phe, 1.02; Val, 1.03; Asp, 1.01; Cys(Bzl), 0.99; Pro, 1.02; Arg, 0.99; Gly, 1.00; $NH_3$, 2.04.

[1-(β-mercapto-β,β-cyclopentamethylenepropionic acid), 2-(O-ethyl)tyrosine, 4-valine]-arginine vasopressin

A solution of protected acylocta-peptide amide (140 mg, 0.095 mmol) in 400 ml of ammonia (dried and redistilled from sodium) was stirred and treated at the boiling point with sodium from a stick of the metal contained in a small-bore glass tube until a light blue color persisted in the solution for 30 s. Dry glacial acetic acid 0.4 ml) was added to discharge the color. The solution was evaporated by passing $N_2$ through the flask. After 5 min, the residue was dissolved in aqueous acetic acid (10%, 50 ml) to which was added 800 ml of water. The solution was treated with 2M ammonium hydroxide solution to give a solution of pH-6.5. An excess of a solution of potassium ferricyanide (0.01M, 16 ml) was added gradually with stirring. The yellow solution was stirred for 10 min more and for 10 min with anion exchange resin (Bio-Rad AG-3, $Cl^-$ form, 10 g damp weight). The suspension was slowly filtered through a bed of resin (50 g damp weight). After washing the bed with aqueous acetic (0.2%, 200 ml), the combined filtrate and washings were lyophylized. The resulting powder (1.63 g) was desalted on a Sephadex® G-15 column (110×2.7 cm) by elution with aqueous acetic acid (50%) at a flow rate 5 ml/h. The eluate was fractionated and monitored for absorbance of 280 nm. The fractions comprising the major peak were pooled and lyophylized. The residue (28 mg) was subjected to gel filtration on a Sephadex® G-15 column (100×1.5 cm). Product was eluted with aqueous acetic acid (0.2M) at a flow rate of 4 ml/h. The peptide was eluted in a single peak (absorbance 280 nm). Lyophylization of the pertinent fractions yielded the vasopressin analog (24 mg, 20.6%). TLC $R_f$(E), 0.31, $R_f$(F), 0.62; $[\alpha]_D=-65.1°$ (C=0.2, 1M AcOH).

Amino acid analysis: Tyr, 1.00; Phe, 1.01; Val, 1.01; Asp, 1.01; Pro, 1.01; Arg, 1.00; Gly, 1.00; $NH_3$, 1.97. Analysis following performic acid oxidation prior to hydrolysis gave a Cys ($O_3H$)-Gly ratio of 1.01:1.00.

[1-(α-mercapto-β,β-cyclopentamethylenepropionic acid], 2-(O-methyl)tyrosine, 4-valine, 8-D-arginine] vasopressin

The peptide intermediate (168 mg, 0.115 mmol) was reduced by sodium in liquid ammonia, reoxidized, deionized, and purified as above to give 49.5 mg of product (35.5%) $R_f$(E), 0.30, $R_f$(F), 0.61; $[\alpha]_D^{23}=-46.4°$ (C=0.4, 1M AcOH).

Amino acid analysis: Tyr, 0.98; Phe, 1.01; Val, 0.98; Asp, 0.99; Pro, 1.03; Arg, 0.98; Gly, 1.00; $NH_3$, 12.1. Analysis following performic acid oxidation prior to hydrolysis gave a Cys($O_3H$)-Gly ratio 1.03:1.00.

[1-(β-mercapto-β,β-cyclopentamethylenepropionic acid), 2-(O-ethyl)tyrosine, 4 valine, 8-D-arginine] vasopressin

The yield of analog from 167 mg (0.113 mmol) of intermediate was 29 mg (20.9%). $T_f$(E), 0.29, $R_f$(F), 0.57; $[\alpha]_D^{23}=-41.1°$ (C=0.3, 1M AcOH).

Amino acid analysis: Tyr, 0.98; Phe, 1.01; Val, 1.03; Asp, 0.99; Pro, 1.03; Arg, 1.02; Gly, 1.00; $NH_3$, 1.98. Analysis following performic acid oxidation prior to hydrolysis gave a Cys ($O_3H$)-Gly ratio 1.01:1.00.

[1-(β-mercapto-β,β-cyclopentamethylenepropionic acid), 2-(O-methyl)tyrosine, 4-valine]-arginine vasopressin

Treatment of the acyl octapeptide (174 mg, 0.119 mmol) as above yielded the 51.5 mg of product (35.6%). $F_f$(E), 0.28, $R_f$(F), 0.60; $[\alpha]_D^{23}=-66.3°$ (C=0.4, 1M AcOH).

Amino acid analysis: Tyr, 0.99; Phe, 1.01; Val, 1.02; Asp, 1.01; Pro, 1.00; Arg, 1.01; Gly, 1.01; $NH_3$, 2.11. Analysis following performic acid oxidation prior to hydrolysis gave a Cys($O_3H$)-Gly ratio 1.03:1.00.

Example 6

[1-(β-mercapto-β,β-cyclopentamethylenepropionic acid), 2-(O-ethyl)tyrosine, 4-valine, 7-(3,4-dehydroproline)] arginine vasopressin

The compound was prepared as in Example 5, using $\Delta^3$-proline instead of proline.

Example 7

[1-(β-mercapto-β,β-cyclopentamethylenepropionic acid, 2-substituted, 4-valine, 7-proline] -(L- and D-) arginine vasopressin

Compounds of this series were prepared as in Example 5. Purity was determined by TLC assay on silica gel in two solvent systems: E. butanol/acetic acid/water (BAW) (4:1:5) or F. butanol/acetic acid/water/pyridine (15:3:3:10). Results were:

| X | Z | $Rf_E$ | $Rf_F$ |
|---|---|---|---|
| D-Tyr | L-Arg | 0.17 | 0.50 |
| D-Phe | L-Arg | 0.17 | 0.52 |
| Gly | L-Arg | 0.15 | 0.48 |
| D-Ala | L-Arg | 0.16 | 0.49 |
| D-Val | L-Arg | 0.17 | 0.49 |
| D-Leu | L-Arg | 0.17 | 0.53 |
| D-Ile | L-Arg | 0.17 | 0.51 |
| D-Arg | L-Arg | 0.08 | 0.30 |
| D-Phe | D-Arg | 0.16 | 0.51 |

Example 8

Antagonism to the vasopressor response was estimated in accordance with Dyckes et al., *J. Med. Chem.*, vol. 17 (1974), page 969 et seq. The values are expressed as $pA_2$ values, defined by Schild et al., *Br. j. Pharmacol.*, vol. 2 (1947), page 189 et seq.

Activity as antidiuretic agonists was determined by intravenous injection of compounds being evaluated into ethanol-anesthesized water-loaded rats in accordance with Sawyer et al., *Endocrinology*, vol. 63 (1958), page 694 et seq.

Antagonistic potencies were determined and expressed as "effective doses" and as $pA_2$ values. The "effective dose" is defined as the dose (in Nanomoles per kilogram) that reduces the response seen from 2x units of agonist injected 20 min after the dose of antagonist to the response with 1x units of agonist. Estimated *in vivo* "$pA_2$" values represent the negative logarithms of the effective doses divided by the estimated volume of distribution (67 nL/kg). Results are given in Table I.

TABLE I

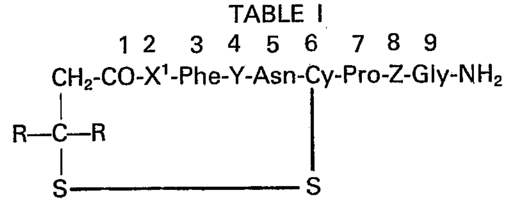

| Peptide | | X¹ | Y | Z | Antivasopressor $pA_2$ | Antidiuretic U/mg |
|---|---|---|---|---|---|---|
| dAVP | $R_1=R_2=H$ | Tyr | Gln | Arg | Agonist | 1745±385 |
| dPAVP | $R_1=R_2=CH_3$ | Tyr | Gln | Arg | 7.45±0.11 | 42±3 |
| $d(CH_2)_5AVP$* | $R_1+R_2=(CH_2)_5$ | Tyr | Gln | Arg | 8.35±0.99 | 0.033±0.005 |
| dVDAVP | $R_1=R_2=H$ | Tyr | Val | D-Arg | 7.03±0.11 | 1230±170 |
| dPVDAVP | $R_1=R_2=CH_3$ | Tyr | Val | D-Arg | 7.82±0.05 | 123±22 |
| $d(CH_2)_5VDAVP$ | $R_1+R_1=(CH_2)_5$ | Tyr | Val | D-Arg | 7.68±0.05 | 0.10±0.02 |
| dTyr(Me)AVP | $R_1=R_2=H$ | Tyr(Me) | Gln | Arg | Agonist | 830±70 |
| dPTyr(Me)AVP | $R_1=R_2=CH_3$ | Tyr(Me) | Gln | Arg | 7.96±0.05 | 3.5±0.5 |
| $d(CH_2)_5Tyr(Me)AVP$* | $R_1+R_2=(CH_2)_5$ | Tyr(Me) | Gln | Arg | 8.62±0.03 | 0.31±0.07 |

*=compound of invention

Results displayed in Table I show that compounds of this invention, particularly [1-(β-mercapto-β,β-cyclopentamethylenepropionic acid), 8-arginine] vasopressin and [1-(β-mercapto-β,β-cyclopentamethylenepropionic acid), 2-(O-methyl)tyrosine, 8-arginine] vasopressin antagonize the vasopressor response to arginine vasopressin and also exhibit a marked reduction in antidiuretic activity.

Example 9

[1-(β-mercapto-β,β-cyclopentamethylenepropionic acid), 2-(O-alkyl)tyrosine, 4-valine, 8-(L- and D-) arginine] vasopressin compounds, evaluated as in Example 8, were weak antidiuretic agonists. They caused an initial submaximal inhibition of urine flow lasting about 10 min, followed by a period of inhibition of responses to ADH lasting 1 to 3 h, depending on the dose. This inhibition was reversible, that is, could be overcome by raising the dose of ADH. Repeated assays permitted estimation of the "effective dose" of each analog, which is defined as the dose which reduces the antidiuretic response to 2x units of ADH injected 20 min after the dose of antagonist to equal the response to 1x units injected prior to the antagonist. The estimated effective doses for these analogs (in nmoles/kg) and antivasopressor activity are given in Table II.

Whereas [1-(β-mercapto-β,β-cyclopentamethylenepropionic acid), 2-(O-alkyl)tyrosine, 4-valine, 8-(L- and D-) arginine] vasopressin compounds were weak antidiuretic agonists, causing an initial submaximal inhibition of urine flow lasting about 10 min, followed by a period of inhibition of responses to ADH lasting 1 to 3 h, the preferred compounds of this invention, indicated by asterisks in the table below, had no antidiuretic agonistic activity.

TABLE II

| Compound | Anti-antidiuretic ED nmoles/Kg | Anti-antidiuretic $pA_2$ | Antivasopressor ED nmoles/Kg | Antivasopressor $pA_2$ |
|---|---|---|---|---|
| $d(CH_2)_5$ D-Tyr VAVP | 2.2±0.2 | 7.51±0.08(4) | 0.29±0.09 | 8.41±0.11(4) |
| $d(CH_2)_5$ D-Phe VAVP | 0.67±0.13* | 8.07±0.09(8) | 0.58±0.04 | 8.06±0.03(4) |
| $d(CH_2)_5[Gly^2]$VAVP | agonist | — | agonist | — |
| $d(CH_2)_5$[D-$Ala^2$]VAVP | agonist | — | 177±31 | 5.79±0.08(4) |
| $d(CH_2)_5$[D-$Val^2$]VAVP | 2.3±0.3* | 7.48±0.06(4) | 27±3 | 6.41±0.05(4) |
| $d(CH_2)_5$[D-$Leu^2$]VAVP | 1.2±0.3* | 7.79±0.12(4) | 26±5 | 6.45±0.09(4) |
| $d(CH_2)_5$[D-$Ile^2$]VAVP | 0.70±0.0* | 7.98±0.05(4) | 8.2±1.4 | 6.94±0.08(5) |
| $d(CH_2)_5$[D-$Arg^2$]VAVP | >90 | >5.9 | ~260 | ~5.4 |
| $d(CH_2)_5$ D-Phe VDAVP | 6.9±1.3 | 7.07±0.10(9) | 0.73 | 7.98±0.07(4) |
| $d(CH_2)_5$Tyr(Me)VDAVP | 15±3 | 6.68±0.11(4) | 0.28±0.05 | 8.44±0.07(8) |
| $d(CH_2)_5$Tyr(Et)VDAVP | 5.7±0.5 | 7.10±0.08(4) | 0.34±0.04 | 8.31±0.05(8) |
| $d(CH_2)_5$Tyr(i-Pr)VDAVP | 8.5±0.07 | 6.88±0.07(4) | 0.28±0.07 | 8.41±0.08(8) |
| $d(CH_2)_5$Tyr(n-Pr)VDAVP | 14±2 | 6.67±0.05(4) | 1.1±0.2 | 7.86±0.10(8) |
| $d(CH_2)_5$Tyr(Me)VAVP | 3.1±0.4 | 7.35±0.06(4) | 0.29±0.06 | 8.32±0.08(4) |
| $d(CH_2)_5$Tyr(Et)VAVP | 1.9±0.2 | 7.57±0.06(4) | 0.49±0.11 | 8.16±0.09(4) |
| $d(CH_2)_5$Tyr(i-Pr)VAVP | 3.6±0.9 | 7.32±0.06(6) | 0.31±0.06 | 8.36±0.09(4) |
| $d(CH_2)_5$Tyr(n-Pr)VAVP | 3.5±0.06 | 7.29±0.07(4) | 0.40±0.04 | 8.22±0.04(4) |
| $d(CH_2)_5$-D-Tyr(Me)VAVP | 1.2±0.3 | 7.77±0.07(6) | 0.23±0.04 | 8.48±0.08(4) |
| $d(CH_2)_5$-D-Tyr(Et)VAVP | 1.1±0.2 | 7.81±0.07(5) | 0.45±0.11 | 8.22±0.12(4) |
| $d(CH_2)_5$-Tyr(Et)-V-$\Delta^3$-$Pro^7$-AVP | 1.5±0.3 | | | |

The preferred compounds of this invention are also more selective than prior art antidiuretic antagonists with respect to antivasopressor potencies, as shown by the ratios of antivasopressor/anti-antidiuretic effective doses:

| Compounds | ED's Antivasopressor / Anti-antidiuretic |
|---|---|
| $d(CH_2)_5$D-Tyr(Et) VAVP | 0.41 |
| $d(CH_2)_5$D-Phe VAVP | 0.87 |
| $d(CH_2)_5$[D-$Val^2$]VAVP | 12 |
| $d(CH_2)_5$[D-$Leu^2$]VAVP | 22 |
| $d(CH_2)_5$[D-$Ile^2$]VAVP | 12 |

Example 10

(a) Antagonism to [1-(β-mercapto-β,β-cyclopentamethylenepropionic acid), 2-(O-alkyl)tyrosine, 4-valine]-(L- and D-)-arginine vasopressin compounds to endogenous ADH was shown by injection of the compound intraperitoneally into conscious rats. Urine was collected for 4 h after the injections. The data in the Table below are the means ±SE of results on groups of 4—6 rats. *$P<0.05$ and **$P<0.005$ are given for differences between the means for rats receiving antagonists and the means for reponses of the same rats injected with solvent only. The mean control urine volume rate for solvent-injected rats was 0.9±0.1 ml/kg per hr and the mean osmolality was 1544±85 mOsm/kg $H_2O$ (n=32).

| | Dose ug/kg | Urine volume ml/kg per hr | Osmolality mOsm/kg $H_2O$ |
|---|---|---|---|
| $d(CH_2)_5$Tyr(Me)VDAVP | 100 | 1.5±0.3 | 1341±428 |
| | 300 | 2.2±0.4* | 961±204** |
| $d(CH_2)_5$Tyr(Et)VDAVP | 30 | 2.8±0.3** | 640±47** |
| | 100 | 9.5±1.8** | 234±25** |
| $d(CH_2)_5$Tyr(Me)VAVP | 10 | 1.1±0.5 | 1303±190 |
| | 30 | 3.4±0.9* | 514±105** |
| $d(CH_2)_5$Tyr(Et)VAVP | 10 | 7.4±1.0** | 316±38** |
| | 30 | 13.3±2.5** | 194±17** |

(b) Responses by intact female rats, weighing 200 to 240 g, to intraperitoneal injections of $d(CH_2)_5$Tyr(Et)VAVP were determined in a block design experiment in which each rat received solvent and both doses of the ADH antagonist. Injections were given at least two days apart. The rats were on water *ad lib.* Injections were made at 11 a.m., after which spontaneously voided urine was collected hourly for four hours.

In Figure 1 is shown osmolality of the urine as a function of time. Urine osmolalities for the control (solvent-injected) were averaged over 2 hour periods owing to infrequency of urination.

In Figure 2 is shown the urine output as a function of time.

In both figures, vertical lines through points indicate SE's.

Example 11

Compounds wherein Tyr at the 2-position is of the D-series and is unetherified (X' is H) are prepared as in Examples 1—5. The compound in which Z- is L-Arg is very active as an antagonist of the antidiuretic acitivity of arginine vasopressin.

The preceding examples can be repeated with similar success by substituting the generically or specifically described reactants and/or operating conditions of this invention for those used in the preceding examples.

**Claims for the Contracting States: BE, CH, DE, FR, GB, IT, LI LU, NL, SE**

1. Compounds of the formula:

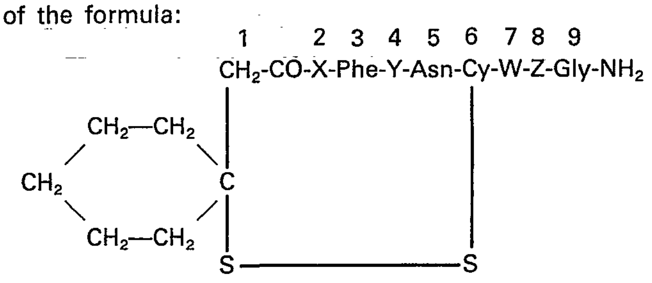

in which:—

X is D-Phe, D-Val, D-Leu, D-Ile, D-Arg, D-norvaline, D-norleucine, D-cyclohexylalanine, D-α-aminobutyric acid, D-threonine, D-methionine, D-Tyr(H), D- or L- Tyr(X') (in which X' is methyl, ethyl, *n*-propyl, isopropyl or butyl) or Tyr(X'') (in which X'' is H, methyl or ethyl);

Y is Val, when X is other than Tyr (X''), or Gln, when X is Tyr(X'');

W is Pro or, when X is Tyr (X'), $\Delta^3$-Pro; and

Z is D- or L-Arg.

2. Compounds as claimed in claim 1 in which X is Tyr-X′; X′ is methyl, ethyl, *n*-propyl, isopropyl or butyl; Tyr is L- or O-; W is Pro or $\Delta^3$-Pro; and Y is Val.

3. [1-(β-Mercapto-β,β-cyclopentamethylenepropionic acid), 2-(O-methyl)tyrosine, 8-arginine] vasopressin.

4. [1-(β-Mercapto-β,β-cyclopentamethylenepropionic acid), 2-(O-methyl)tyrosine, 4-valine, 8-(L- or D-) arginine] vasopressin.

5. [1-(β-(β-Mercapto-β,β-cyclopentamethylenepropionic acid), 2-(O-ethyl)tyrosine, 4-valine 8-(L- or D-) arginine] vasopressin.

6. [1-(β-Mercapto-β,β-cyclopentamethylenepropionic acid), 2-(O-methyl)-D-tyrosine, 4-valine 8-(L- or D-(arginine] vasopressin.

7. [1-(β-Mercapto-β,β-cyclopentamethylenepropionic acid), 2-(O-ethyl)-D-tyrosine, 4-valine 8-(L- or D-) arginine] vasopressin.

8. [1-(β-Mercapto-β,β-cyclopentamethylenepropionic acid), 2-(O-isopropyl)tyrosine, 4-valine, 8-(L- or D-) arginine] vasopressin.

9. [1-(β-Mercapto-β,β-cyclopentamethylenepropionic acid), 2-(O-*n*-propyl)tyrosine, 4-valine, 8-(L- or D-)arginine] vasopressin.

10. [1-(β-Mercapto-β,β-cyclopentamethylenepropionic acid), 2-(O-ethyl)tyrosine, 4-valine, 7-(3,4-dehydroproline)] arginine vasopressin.

11. Compounds as claimed in claim 1 in which X is D-Phe, D-Val, D-Leu, D-Ile, D-Arg, D-norvaline, D-norleucine, D-cyclohexylalanine, D-α-aminobutyric acid, D-threonine, or D-methionine; W is proline, Y is Val and Z is D- or L-arginine.

12. Compounds as claimed in claim 11 in which X is D-Phe, D-Val, D-Leu or D-Ile.

13. Compounds as claimed in claim 11 or claim 12 in which Z is L-Arg.

14. [1-(β-Mercapto-β,β-cyclopentamethylenepropionic acid), 2-D-phenylalanine, 4-valine]-arginine vasopressin.

15. [1-(β-Mercapto-β,β-cyclopentamethylenepropionic acid), 2-D-valine, 4-valine]-arginine vasopressin.

16. [1-(β-Mercapto-β,β-cyclopentamethylenepropionic acid), 2-D-leucine, 4-valine]-arginine vasopressin.

17. [1-(β-Mercapto-β,β-cyclopentamethylenepropionic acid, 2-D-isoleucine, 4-valine]-arginine vasopressin.

18. [1-(β-Mercapto-β,β-cyclopentamethylenepropionic acid, 2-D-phenylalanine, 4-valine, 8-D-arginine] vasopressin.

19. Compounds as claimed in claim 1 in which X is Tyr-X″, Y is Gln, W is Pro and Z is L- or D-Arg.

20. Compounds as claimed in claim 1 in which X is Tyr-H, Tyr is D-Tyr, Y is Val and Z is D- or L-Arg.

21. A pharmaceutical composition comprising a compound as claimed in claim 1 in association with a pharmaceutical carrier or diluent.

22. A pharmaceutical composition as claimed in claim 21 comprising a compound as claimed in any one of claims 2—10 in association with a pharmaceutical carrier or diluent.

23. A pharmaceutical composition as claimed in claim 21 comprising a compound as claimed in claim 11 in association with a pharmaceutical carrier or diluent.

24. A pharmaceutical composition as claimed in claim 21 comprising a compound as claimed in claim 19 in association with a pharmaceutical carrier or diluent.

25. A pharmaceutical composition as claimed in any one of claims 21—24 in a form suitable for parenteral administration.

**Claim for the Contracting State: AT**

1. A process for the preparation of a compound of the formula:

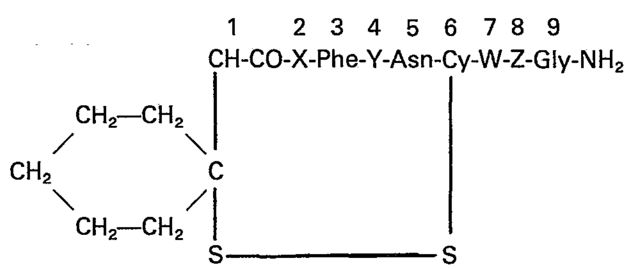

which comprises reducing a compound of the formula:

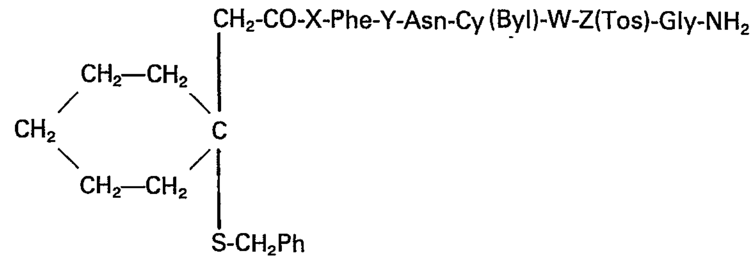

(in which X, Y, W and Z have the meanings defined above) with sodium in liquid ammonia and oxidatively cyclizing the resulting disulthydryl compound with potassium forricyanide.

**Patentansprüche für die Vertragsstaaten: BE, CH, DE, FR, GB, IT, LI, LU, NL, SE**

1. Verbindungen der Formel:

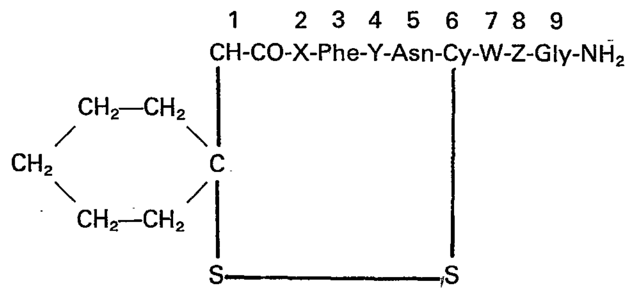

worin:
X für D-Phe, D-Val, D-Leu, D-Ile, D-Arg, D-Norvalin, D-Norleucin, D-Cyclohexylalanin, D-α-Aminobuttersäure, D-Threonin, D-Methionin, D-Tyr(H), D- oder L-Tyr(X') (wobei X' Methyl, Ethyl, *n*-Propyl, Isopropyl oder Butyl bedeutet) oder Tyr(X'') (wobei X'' H, Methyl oder Ethyl bedeutet);
Y für Val, wenn X eine andere Bedeutung als Tyr(X'') hat, oder Gln, wenn X Tyr(X'') bedeutet;
W für Pro oder, wenn X Tyr(X') bedeutet, $\Delta^3$-Pro; und
Z für D- oder L-Arg steht.

2. Verbindungen nach Anspruch 1, worin X für Tyr-X'; X' für Methyl, Ethyl, *n*-Propyl, Isopropyl oder Butyl; Tyr für L- oder D-; W für Pro oder $\Delta^3$-Pro; und Y für Val steht.

3. [1-(β-Mercapto-β,β-cyclopentamethylenpropionsäure), 2-(O-methyl)tyrosin, 8-arginin]vasopressin.

4. [1-(β-Mercapto-β,β-cyclopentamethylenpropionsäure), 2-(O-methyl)tyrosin, 4 valin, 8-(L- oder D-)arginin]vasopressin.

5. [1-(β-Mercapto-β,β-cyclopentamethylenpropionsäure), 2-(O-ethyl)tyrosin, 4-valin, 8-(L- oder D-)arginin]vasopressin.

6. [1-(β-Mercapto-β,β-cyclopentamethylenpropionsäure), 2-(O-methyl)-D-tyrosin, 4-valin, 8-(L- oder D-)arginin]vasopressin.

7. [1-(β-Mercapto-β,β-cyclopentamethylenpropionsäure), 2-(O-ethyl)-D-tyrosin, 4-valin, 8-(L- oder D-)arginin]vasopressin.

8. [1-(β-Mercapto-β,β-cyclopentamethylenpropionsäure), 2-(O-isopropyl)tyrosin, 4-valin, 8-(L- oder D-)arginin]vasopressin.

9. [1-(β-Mercapto-β,β-cyclopentamethylenpropionsäure),2-(O-*n*-propyl)tyrosin, 4-valin, 8-(L- oder D-)arginin]vasopressin.

10. [1-(β-Mercapto-β,β-cyclopentamethylenpropionsäure), 2-(O-ethyl)tyrosin, 4-valin, 7-(3,4-dehydroprolin)arginin]vasopressin.

11. Verbindungen nach Anspruch 1, worin X für D-Phe, D-Val, D-Leu, D-Ile, D-Arg, D-Norvalin, D-Norleucin, D-Cyclohexylalanin, D-α-Aminobuttersäure, D-Threonin oder D-Methionin; W für Prolin, Y für Val und Z für D- oder L-Arginin steht.

12. Verbindungen nach Anspruch 11, worin X für D-Phe, D-Val, D-Leu oder D-Ile steht.

13. Verbindungen nach Anspruch 11 oder Anspruch 12, worin Z für L-Arg steht.

14. [1-(β-Mercapto-β,β-cyclopentamethylenpropionsäure), 2-D-phenylalanin, 4-valin]-arginin-vasopressin.

15. [1-(β-Mercapto-β,β-cyclopentamethylenpropionsäure), 2-D-valin, 4-valin]-argininvasopressin.

16. [1-(β-Mercapto-β,β-cyclopentamethylenpropionsäure), 2-D-leucin, 4-valin]-argininvasopressin.

17. [1-(β-Mercapto-β,β-cyclopentamethylenpropionsäure), 2-D-isoleucin, 4-valin]-argininvasopressin.

18. [1-(β-Mercapto-β,β-cyclopentamethylenpropionsäure), 2-D-phenylalanin, 4-valin, 8-D-arginin]-vasopressin.

19. Verbindungen nach Anspruch 1, worin X für Tyr-X'', Y für Gln, W für Pro und Z für L- oder D-Arg steht.

20. Verbindungen nach Anspruch 1, worin X für Tyr-H, Tyr für D-Tyr, Y für Val und Z für D- oder L-Arg steht.

21. Pharmazeutische Zusammensetzung, dadurch gekennzeichnet, daß sie eine Verbindung nach Anspruch 1 in Assoziation mit einer pharmazeutischen Trägersubstanz oder einem Verdünnungsmittel enthält.

22. Pharmazeutische Zusammensetzung nach Anspruch 21, dadurch gekennzeichnet, daß sie eine Verbindung nach einem der Ansprüche 2 bis 10 in Assoziation mit einer pharmazeutischen Trägersubstanz oder einem Verdünnungsmittel enthält.

23. Pharmazeutische Zusammensetzung nach Anspruch 21, dadurch gekennzeichnet, daß sie eine Verbindung nach Anspruch 11 in Assoziation mit einer pharmazeutischen Trägersubstanz oder einem Verdünnungsmittel enthält.

24. Pharmazeutische Zusammensetzung nach Anspruch 21, dadurch gekennzeichnet, daß sie eine Verbindung nach Anspruch 19 in Assoziation mit einer pharmazeutischen Trägersubstanz oder einem Verdünnungsmittel enthält.

25. Pharmazeutische Zusammensetzung nach einem der Ansprüche 21 bis 24 in einer für die parenterale Applikation geeigneten Form.

**Patentanspruch für den Vertragsstaat: AT**

1. Verfahren zur Herstellung einer Verbindung der Formel:

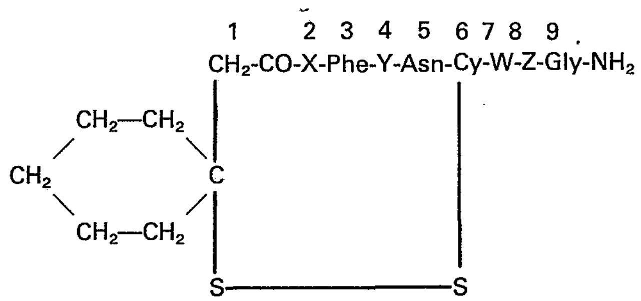

dadurch gekennzeichnet, daß eine Verbindung der Formel:

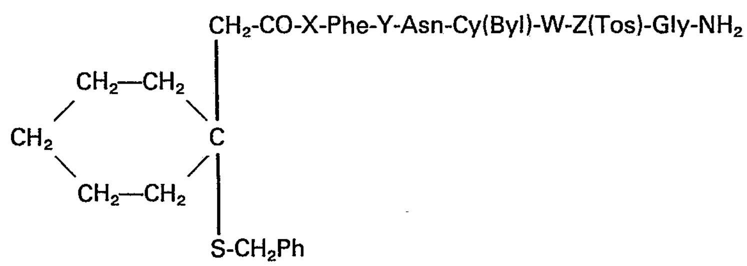

(in welcher X, Y, W und Z die oben angeführten Bedeutungen haben), mit Natrium in flüssigem Ammoniak reduziert und die sich ergebende Disulfhydrylverbindung mit Kaliumferrocyanid oxydativ cyclisiert wird.

**Revendications pour les États Contractants: BE, CH, DE, FR, GB, IT, LI, LU, NL, SE**

1. Composés de la formule

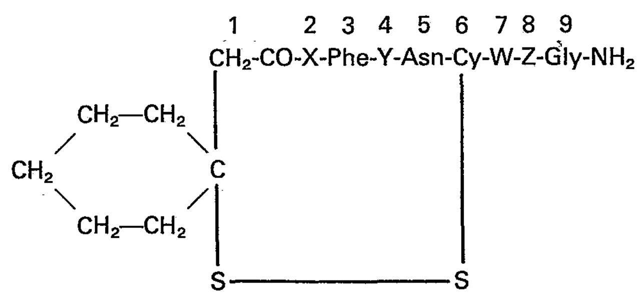

dans lesquels:

X est D-Phe, D-Val, D-Leu, D-Ile, D-Arg, D-norvaline, D-norleucine, D-cyclohexylalanine, D-acide

aminobutyrique alpha, D-threonine, D-methionine, D-Tyr(H), D- ou L-Tyr(X') (X' étant méthyle, éthyle, propyle-*n*, isopropyle ou butyle) ou Tyr (X'') (X'') étant H, méthyle ou éthyle);

Y est Val quand X est autre que Tyr (X''), ou Gln, quand X est Tyr (X'');

W est Pro ou, lorsque X est Tyr (X') $\Delta^3$-Pro; et

Z est D- ou L-Arg.

2. Composés selon la revendication 1, dans lesquels X est Tyr-X'; X' est méthyle, éthyle, propyle-*n*, isopropyle ou butyle; Tyr est L- ou D-; W est Pro ou $\Delta^3$-Pro; et Y est Val.

3. Vasopressine [1-(β-mercapto-β, acide cyclopentaméthylènepropionique-β), 2-(O-méthyle)tyrosine, 8-arginine].

4. Vasopressine [1-(β-mercapto-β, acide cyclopentaméthylènepropionique-β), 2-(O-méthyl)tyrosine, 4-valine 8-(L- ou D-) arginine].

5. Vasopressine [1-(β-mercapto-β, acide cyclopentaméthylènepropionique-β), 2-(O-éthyle)tyrosine, 4-valine 8-(L- ou D-) arginine].

6. Vasopressine [1-(β-mercapto-β, acide cyclopentaméthylènepropionique-β), 2-(O-méthyle)-D-tyrosine, 4-valine 8-(L- ou D-) arginine].

7. Vasopressine [1-(β-mercapto-β, acide cyclopentaméthylènepropionique-β); 2-(O-éthyle)-D-tyrosine, 4-valine 8-(L- ou D-) arginine].

8. Vasopressine [1-(β-mercapto-β, acide cyclopentaméthylènepropionique-β), 2-(O-isopropyle)tyrosine, 4-valine, 8-(L- ou D-) arginine].

9. Vasopressine [1-(β-mercapto-β, acide cyclopentaméthylènepropionique-β), 2-(O-*n*-propyle)tyrosine, 4-valine, 8-(L- ou D-) arginine].

10. Vasopressine [1-(β-mercapto-β, acide cyclopentaméthylènepropionique-β), 2-(O-éthyle)tyrosine, 4-valine, 7-(3,4-déhydroproline)]arginine.

11. Composés selon la revendication 1, dans lesquels X est D-Phe, D-Val, D-Leu, D-Ile, D-Arg, D-norvaline, D-norleucine, D-cyclohexylalanine, D-acide aminobutyrique alpha, D-threonine, ou D-methionine; W est proline, Y est Val et Z est D- ou L-arginine.

12. Composés selon la revendication 11, dans lesquels X est D-Phe, D-Val, D-Leu ou D-Ile.

13. Composés selon la revendication 11 ou 12, dans lesquels Z est L-Arg.

14. Vasopressine [1-(β-mercapto-β, acide cyclopentaméthylènepropionique-β), 2-D-phenylalanine, 4-valine]arginine.

15. Vasopressine [1-(β-mercapto-β, acide cyclopentaméthylènepropionique-β), 2-D-valine, 4-valine]-arginine.

16. Vasopressine [1-(β-mercapto-β, acide cyclopentaméthylènepropionique-β), 2-D-leucine, 4-valine]-arginine.

17. Vasopressine [1-(β-mercapto-β, acide cyclopentaméthylènepropionique-β), 2-D-isoleucine, 4-valine]-arginine.

18. Vasopressine [1-(β-mercapto-β, acide cyclopentaméthylènepropionique-β), 2-D-phénylalanine, 4-valine, 8-D-arginine].

19. Composés selon la revendication 1, dans lesquels X est Tyr-X'', Y est Gln, W est pro et Z est L- ou D-Arg.

20. Composés selon la revendication 1, dans lesquels X est Tyr-H, Tyr est D-Tyr, Y est Val et Z est D- ou L-Arg.

21. Une composition pharmaceutique comprenant un composé selon la revendication 1, en association avec un excipient ou diluant pharmaceutique.

22. Une composition pharmaceutique selon la revendication 21, comprenant un composé selon une quelconque des formules des revendications 2, à 10 en association avec un excipient ou diluant pharmaceutique.

23. Une composition pharmaceutique selon la revendication 21, comprenant un composé selon la revendication 11 en association avec un excipient ou diluant pharmaceutique.

24. Une composition pharmaceutique selon la revendication 21, comprenant un composé selon la revendication 19, en association avec un excipient ou diluant pharmaceutique.

25. Une composition pharmaceutique selon une quelconque des revendications 21—24, sous une forme appropriée pour administration parentérale.

**Revendication pour l'Etat Contractant: AT**

1. Un procédé pour la préparation d'un composé de la formule:

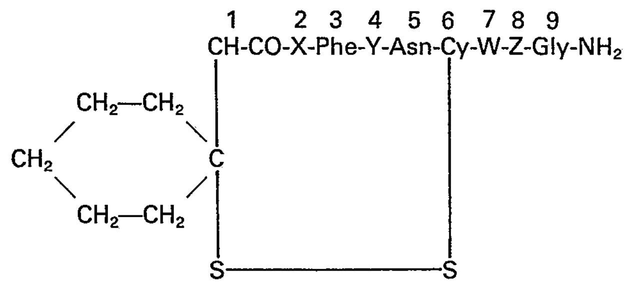

qui comprend la réduction d'un composé de la formule:

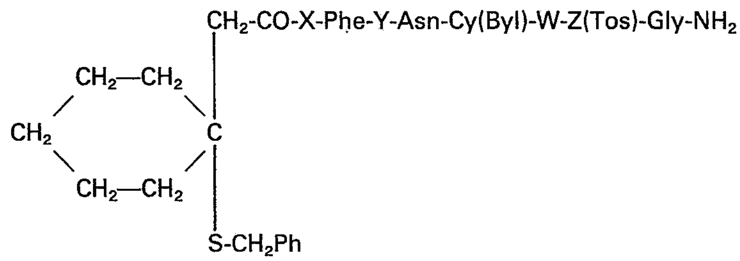

dans lesquels:

X est D-Phe, D-Val, D-Leu, D-Ile, D-Arg, D-norvaline, D-norleucine, D-cyclohexylalanine, D-acide aminobutyrique alpha, D-threonine, D-methionine, D-Tyr(H) D- ou L-Tyr (X') (X' étant méthyle, éthyle, propyle-*n*, isopropyle ou butyle) ou Tyr (X'') (X'') étant H, méthyle ou éthyle);

Y est Val quand X est autre que Tyr (X''), ou Gln, quand X est Tyr (X'');

W est Pro ou, lorsque X est Tyr (X') $\Delta^3$-Pro; et

Z est D- ou L-Arg.

avec du sodium dans l'ammoniaque liquide et cyclisant de manière oxydante le composé disulthydryl résultant avec le forricyanide de potassium.

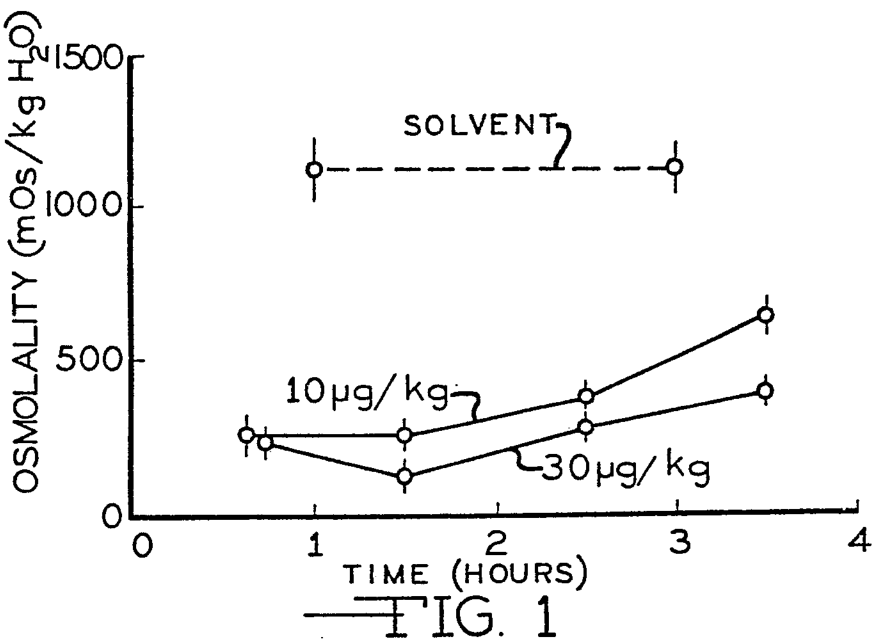
OSMOLALITY (mOs/kg H2O)
1500
1000
500
0
SOLVENT
10µg/kg
30µg/kg
0
1
2
3
4
TIME (HOURS)

FIG. 1

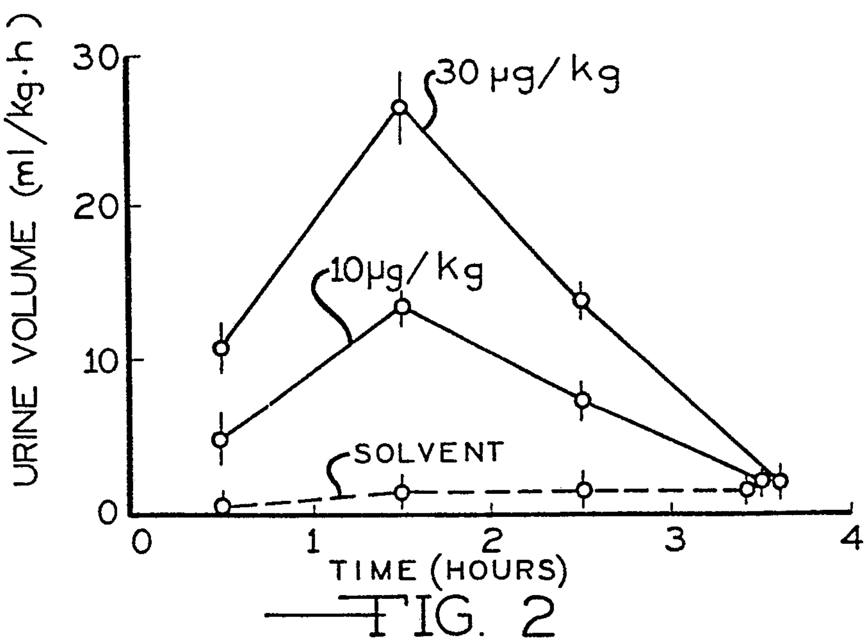
URINE VOLUME (ml/kg·h)
30
20
10
0
30 µg/kg
10µg/kg
SOLVENT
0
1
2
3
4
TIME (HOURS)

FIG. 2